# EUROPEAN PATENT SPECIFICATION

(11) **EP 4 037 755 B1**
(45) Date of publication and mention of the grant of the patent: **17.07.2024**
(21) Application number: 20792759.1
(22) Date of filing: 30.09.2020
(51) Int. Cl.: A61N 1/04, A61N 1/36, A61B 5/0531, A61B 5/259, A61B 5/266, A61B 5/389, A61B 5/00

(54) **BODY ELECTRODE AND BODY ELECTRODE UNIT**
KÖRPERELEKTRODE UND KÖRPERELEKTRODENEINHEIT
ÉLECTRODE CORPORELLE ET UNITÉ D'ÉLECTRODE CORPORELLE

(30) Priority: 04.10.2019 JP 2019183802; 18.09.2020 JP 2020157337
(43) Date of publication of application: 10.08.2022
(73) Proprietor: Nihon Kohden Corporation, Shinjuku-ku Tokyo 161-8560 (JP)
(72) Inventor: ODAKA, Ryugo, Tokorozawa-shi, Saitama 359-0037 (JP); NISHIWAKI, Shigehiro, Tokorozawa-shi, Saitama 359-0037 (JP); YOSHIHARA, Hiroshi, Tokorozawa-shi, Saitama 3590037 (JP); IWATA, Shunji, Tokorozawa-shi, Saitama 3590037 (JP)
(74) Representative: Grünecker Patent- und Rechtsanwälte PartG mbB
(86) International application number: PCT/JP2020/037346
(87) International publication number: WO 2021/066077

(56) References cited:
- JP-A- H09 313 454
- US-A1- 2002 183 647
- US-A1- 2007 129 771
- US-A1- 2010 210 965
- US-A1- 2014 235 991

## Description

### Technical Field

The presently disclosed subject matter relates to a body electrode and body electrode unit configured to be used in measurement of physiological information.

### Background

A related art body electrode is provided in a state where a face on which an adhesive gel is disposed is attached to a release sheet. When using the body electrode, the body electrode is peeled off from the release sheet, the face on which the adhesive gel is disposed is attached to a body (a subject).

The body electrode may have a plurality of electrodes mounted on the surface of a substrate sheet (see, e.g., JPH09-313454A). In such a body electrode, the plurality of electrodes are mounted on the one substrate sheet while being separated from one another. Therefore, the body electrode can be attached to a body while maintaining predetermined intervals.

In such a body electrode, however, the plurality of electrodes are pattern-printed to the one substrate sheet. That is, the electrodes are mounted at fixed positions, respectively. With this configuration, when attaching the body electrode to a hand of a subject, for example, the body electrode may not be attached to a desired position depending on the size of the hand of the subject.
US 2014/0235991 discloses an electrode system for use with neuromuscular monitoring systems.

### Summary

The invention is defined by the independent claim 1. Illustrative aspects of the presently disclosed subject matter provide a body electrode and body electrode unit in which an electrode can be attached to a desired position.

According to an aspect of the presently disclosed subject matter, a body electrode includes a first electrode configured to stimulate a muscle of a body, a second electrode, and a third electrode. The second electrode and the third electrode are configured to detect a physiological signal from the muscle that is stimulated by the first electrode. The body electrode also includes a first connection portion arranged between the first electrode and the second electrode, and a second connection portion arranged between the third electrode and one of the first electrode and the second electrode. The first connection portion has at least one first direction changing part configured to change a direction in which the first connection portion extends, such that at least one of a distance and an angle between the first electrode and the second electrode is adjustable.

According to another aspect of the presently disclosed subject matter, a body electrode unit includes the body electrode and a release sheet to which the body electrode is attached.

### Brief Description of Drawings

[fig.1A] FIG. 1A is a view illustrating a front side a body electrode.
[fig.1B] FIG. 1B is a view illustrating a back side a body electrode.
[fig.2A] FIG. 2A is a view illustrating a body electrode unit.
[fig.2B] FIG. 2B is another view illustrating a body electrode unit.
[fig.3] FIG. 3 is a view illustrating a lateral side of the body electrode unit.
[fig.4A] FIG. 4A is a view illustrating how the body electrode unit is used.
[fig.4B] FIG. 4B is another view illustrating how the body electrode unit is used.
[fig.4C] FIG. 4C is another view illustrating how the body electrode unit is used.
[fig.4D] FIG. 4D is another view illustrating how the body electrode unit is used.
[fig.5A] FIG. 5A is another view illustrating how the body electrode unit is used.
[fig.5B] FIG. 5B is another view illustrating how the body electrode unit is used.
[fig.5C] FIG. 5C is another view illustrating how the body electrode unit is used.
[fig.5D] FIG. 5D is another view illustrating how the body electrode unit is used.
[fig.6A] FIG. 6A is a view illustrating an example of first and second connection portions.
[fig.6B] FIG. 6B is a view illustrating another example of first and second connection portions.
[fig.6C] FIG. 6C is a view illustrating another example of first and second connection portions.
[fig.7A] FIG. 7A is a view illustrating an example a neutral electrode.
[fig.7B] FIG. 7B is another view illustrating the example the neutral electrode.
[fig.8] FIG. 8 is a view illustrating a potential waveform of an active electrode.
[fig.9] FIG. 9 is a view illustrating another example the neutral electrode.
[fig.10] FIG. 10 is a view illustrating yet another example the neutral electrode.
[fig.11A] FIG. 11A is a view illustrating yet another example the neutral electrode.
[fig.11B] FIG. 11B is a view illustrating yet another example of the neutral electrode.

### Description of Embodiments

Hereinafter, embodiments of the presently disclosed subject matter will be described with reference to the drawings.

### Structure of Body Electrode 1

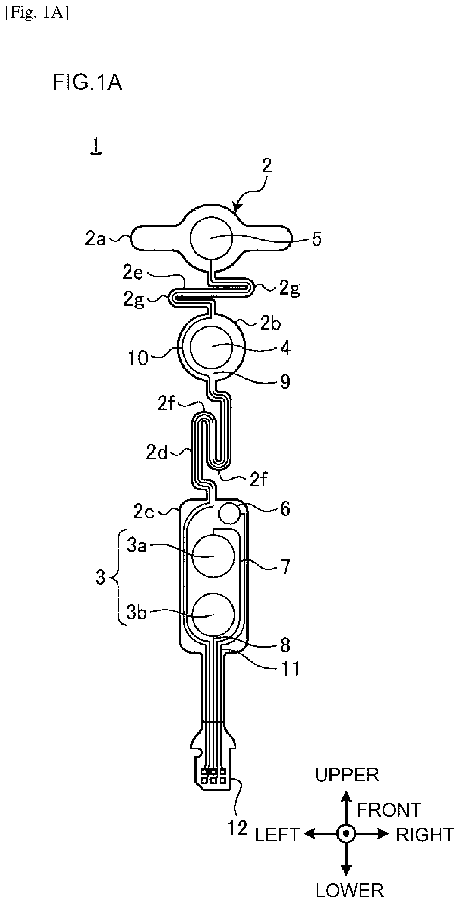

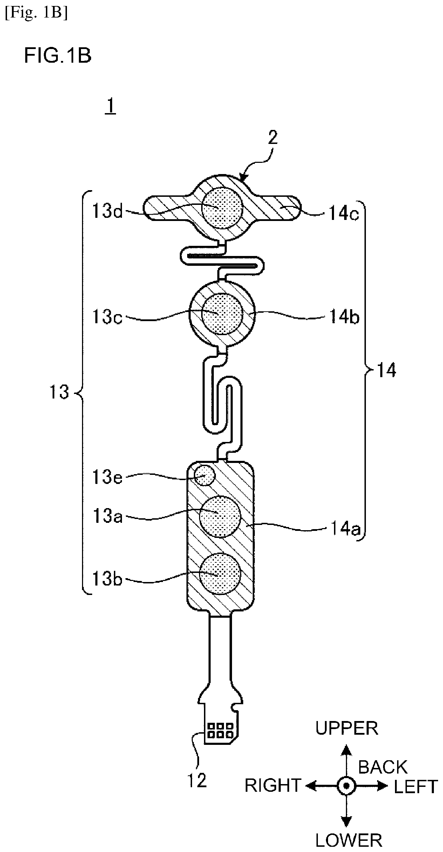

First, a structure of a body electrode 1 will be described with reference to FIGS. 1A and 1B. FIG. 1A is a front view of the body electrode 1, and FIG. 1B is a back view of the body electrode 1.

In this example, the body electrode 1 is used to stimulate a nerve leading to a muscle of a body (a subject to which the body electrode 1 is attached), and to monitor the degree of relaxation of the reacting muscle based on a physiological signal of the muscle. For example, the body electrode 1 is used in the case where the degree of relaxation of a muscle of a body is monitored by the TOF (Train of Four) method in which muscle stimulation is successively performed four times every 0.5 seconds, and the process of four successive muscle stimulations is repeated every 15 seconds.

A stimulation electrode 3, an active electrode 4, a reference electrode 5, and a neutral electrode 6 are mounted on a base 2. The base 2 includes an upper island portion 2a on which the reference electrode 5 is mounted, an intermediate island portion 2b on which the active electrode 4 is mounted, a lower island portion 2c on which the stimulation electrode 3 and the neutral electrode 6 are mounted, a first connection portion 2d provided between the intermediate island portion 2b and the lower island portion 2c, and a second connection portion 2e provided between the upper island portion 2a and the intermediate island portion 2b.

The first connection portion 2d has a first direction changing part 2f configured to change the direction in which the first connection portion 2d extends. Since the first connection portion 2d has the first direction changing part 2f, at least one of the distance and the angle between the stimulation electrode 3 and the active electrode 4 is adjustable.

The second connection portion 2e has a second direction changing part 2g configured to change the direction in which the second connection portion 2e extends. Since the second connection portion 2e has the second direction changing part 2g, at least one of the distance and the angle between the active electrode 4 and the reference electrode 5 is adjustable.

The first direction changing part 2f is configured to change the direction in which the first connection portion 2d extends in the longitudinal direction of the body electrode 1. The second direction changing part 2g is configured to change the direction in which the second connection portion 2e extends in the lateral direction of the body electrode 1. That is, the first direction changing part 2f and the second direction changing part 2g are configured to change the direction in which the first connection portion 2d extends and the direction in which the second connection portion 2e extends in different directions, respectively.

The first direction changing part 2f is configured to change the direction in which the first connection portion 2d extends in the longitudinal direction of the body electrode 1 to prevent, when attaching the body electrode 1 to the subject, the first connection portion 2d from rising up and from contacting the wrist of the subject to stimulate the sensation of pain.

The second direction changing part 2g is configured to change the direction in which the second connection portion 2e extends in the lateral direction of the body electrode 1 to prevent, when attaching the body electrode 1 the subject, the second connection portion 2e from rising up and from contacting the hypothenar to stimulate the sensation of pain.

The stimulation electrode 3 is disposed at a position closer to a connector 12 in the longitudinal direction of the body electrode 1 as compared with the reference electrode 5, and is configured to apply electrical stimulation to a nerve leading to a muscle of the body (for example, the ulnar nerve). The stimulation electrode 3 includes a negative electrode 3a and a positive electrode 3b.

The active electrode 4 is disposed between the stimulation electrode 3 and the reference electrode 5, and provided to detect a physiological signal from a muscle that responds to the electrical stimulation applied by the stimulation electrode 3. In the embodiment, the active electrode 4 is configured by a cathode. Here, the active electrode 4 is mounted corresponding to, for example, the abductor digiti minimi muscle.

The reference electrode 5 is disposed at a position farther from the connector 12 in the longitudinal direction of the body electrode 2 with respect to the stimulation electrode 3 and the active electrode 4, and is configured to detect a physiological signal from a muscle that responds to the electrical stimulation applied by the stimulation electrode 3. In the embodiment, the reference electrode 5 is a positive electrode. Here, the reference electrode 5 is mounted corresponding to, for example, the abductor digiti minimi muscle.

The neutral electrode 6 is provided to block noises flowing through the body electrode 1. The neutral electrode 6 is disposed proximal to the negative electrode 3a. More specifically, the neutral electrode 6 is mounted on an upper side of the negative electrode 3a. In other words, the neutral electrode 6 is mounted in a direction toward the active electrode 4 from the negative electrode 3a, or a direction toward the active electrode 4 from the connector 12. Furthermore, it can be said also that the neutral electrode 6 is mounted in a region between the negative electrode 3a and the active electrode 4. Therefore, noises that are generated in the case where the negative electrode 3a and the active electrode 4 are close to each other are easily blocked.

A negative electrode wiring part 7 is provided to connect the negative electrode 3a and the connector 12 to each other. Based on an operation performed on an electric stimulator (not illustrated), therefore, the negative electrode 3a applies stimulation to the body through the negative electrode wiring part 7.

A positive electrode wiring part 8 is provided to connect the positive electrode 3b and the connector 12 to each other. Based on an operation performed on the electric stimulator, therefore, the positive electrode 3b applies stimulation to the body through the positive electrode wiring part 8.

An active electrode wiring part 9 is provided to connect the active electrode 4 and the connector 12 to each other. Based on a detection of a physiological signal of the living bode, therefore, the active electrode 4 outputs the physiological signal to the electric stimulator through the active electrode wiring part 9. A part of the active electrode wiring part 9 is arranged in the first connection portion 2d.

A reference electrode wiring part 10 is provided to connect the reference electrode 5 and the connector 12 to each other. Based on a detection of a physiological signal of the living bode, therefore, the reference electrode 5 outputs the physiological signal to the electric stimulator through the reference electrode wiring part 10. A part of the reference electrode wiring part 10 is arranged in the second connection portion 2e.

A neutral electrode wiring part 11 is provided to connect the neutral electrode 6 and the connector 12 to each other.

The connector 12 is provided to connect the wiring parts respectively connected to the electrodes. Specifically, the negative electrode wiring part 7 connected to the negative electrode 3a, the positive electrode wiring part 8 connected to the positive electrode 3b, the active electrode wiring part 9 connected to the active electrode 4, the reference electrode wiring part 10 connected to the reference electrode 5, and the neutral electrode wiring part 11 connected to the neutral electrode 6 are connected to the connector 12. The connector 12 is further connected to the electric stimulator. Spare connecting parts that are not connected to any wiring part are disposed in the connector 12.

An adhesive gel 13 is provided to attach the electrodes to the body, and covers the back sides of the electrodes. Specifically, the adhesive gel 13 includes a negative electrode adhesive gel 13a that covers the back side of the negative electrode 3a, a positive electrode adhesive gel 13b that covers the back side of the positive electrode 3b, an active electrode adhesive gel 13c that covers the back side of the active electrode 4, a reference electrode adhesive gel 13d that covers the back side of the reference electrode 5; and a neutral electrode adhesive gel 13e that covers the back side of the neutral electrode 6.

An adhesive tape 14 is provided to attach the body electrode 1 to a release sheet 100 that will be described later, and has an adhesiveness. As illustrated in FIG. 1B, the adhesive tape 14 has a configuration including: a stimulation electrode adhesive tape 14a that is disposed on the back side of the lower island portion 2c, and in the peripheries of the negative electrode adhesive gel 13a, the positive electrode 3b, and the neutral electrode adhesive gel 13e; an active electrode adhesive tape 14b that is disposed on the back side of the intermediate island portion 2b, and in the periphery of the active electrode adhesive gel 13c; and a reference electrode adhesive tape 14c that is disposed on the back side of the upper island portion 2a, and in the periphery of the reference electrode adhesive gel 13d.

In place of the adhesive tape 14, for example, a configuration in which paste is attached to the upper island portion 2a, the intermediate island portion 2b, and the lower island portion 2c, and which is attached to the release sheet 100 may be employed. That is, it is requested only to perform a process so as to provide the upper island portion 2a, the intermediate island portion 2b, and the lower island portion 2c with an adhesiveness. Here, "adhesiveness" means possession of a viscosity of a degree at which the portions are stuck and held to the release sheet 100 or the skin of the subject.

An adhesive tape is not disposed on the back side of the first connection portion 2d, and that of the second connection portion 2e. That is, the back side of the first connection portion 2d, and that of the second connection portion 2e do not have an adhesiveness. When the stimulation electrode adhesive tape 14a, the active electrode adhesive tape 14b, and the reference electrode adhesive tape 14c are to be attached to the subject, therefore, a situation where the tapes are attached to unintended positions can be prevented from occurring.

### Body Electrode Unit U

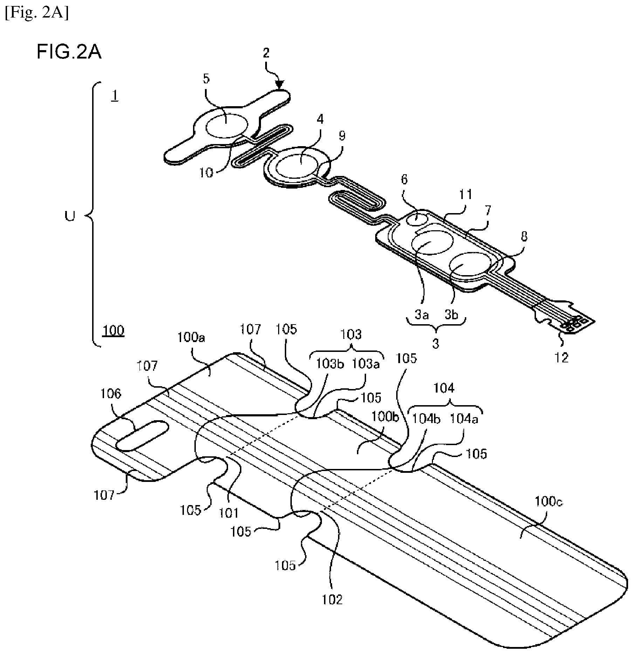

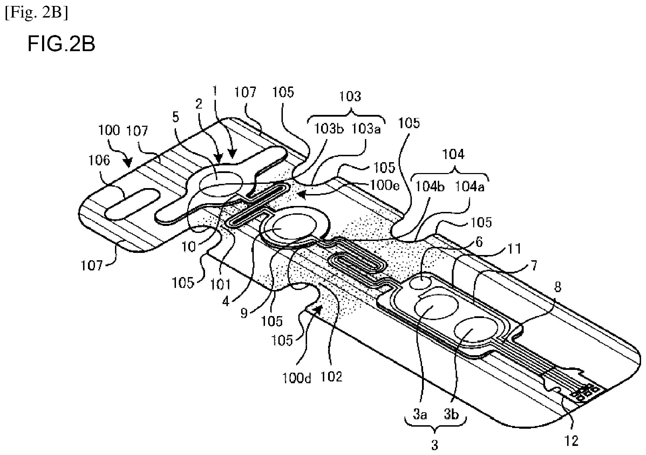

Referring to FIGS. 2A and 2B, next, a body electrode unit U will be described. FIG. 2A is an exploded perspective view of the body electrode unit U, and FIG. 2B is a perspective view of the body electrode unit U.

As illustrated in FIG. 2A, the body electrode unit U has the body electrode 1 that has been described with reference to FIGS. 1A and 1B, and the release sheet 100.

The release sheet 100 is a sheet to which the body electrode 1 is to be attached, and can transmit light. During a process for producing the body electrode unit U, therefore, it is easily check whether the adhesive gel 13 is provided cover the back sides of the electrodes.

The release sheet 100 has: an upper sheet portion 100a to which the reference electrode adhesive tape 14c of the upper island portion 2a is to be attached; an intermediate sheet portion 100b to which the active electrode adhesive tape 14b of the intermediate island portion 2b is to be attached; and a lower sheet portion 100c to which the stimulation electrode adhesive tape 14a of the lower island portion 2c is to be attached. The release sheet 100 includes a first processed part 101, a second processed part 102, a first notch 103, a second notch 104, rounded parts 105, a positioning hole 106, and patterned portions 107.

A process for facilitating separation and/or bending of the upper sheet portion 100a and the intermediate sheet portion 100b is applied to the first processed part 101.

A process for facilitating separation and/or bending of the intermediate sheet portion 100b and the lower sheet portion 100c is applied to the second processed part 102.

In the embodiment, a perforation process is performed in the first processed part 101 and the second processed part 102 as a mode in which the release sheet 100 can be separated and/or bent. However, the mode is not limited to this, and any mode may be used in so far as it can separate and/or bend the release sheet 100. For example, a half-cut process in which a part of the thickness of the release sheet 100 is cut may be applied to the first processed part 101 and the second processed part 102. In the thickness (e.g., about 75 µm) of the release sheet 100, specifically, the cutting is performed at a predetermined thickness (e.g., about 50 µm) that is smaller than the thickness of the release sheet 100, thereby enabling the release sheet 100 to be separated and/or bent.

In the embodiment, the first processed part 101 may be disposed at any position in so far as it is in a first area 100d that, in the case where the body electrode 1 is attached to the release sheet 100, is between the lower end of the intermediate island portion 2b and the upper end of the lower island portion 2c. In the same or similar manner, the second processed part 102 may be disposed at any position in so far as it is in a second area 100e that, in the case where the body electrode 1 is attached to the release sheet 100, is between the lower end of the upper island portion 2a and the upper end of the intermediate island portion 2b.

The first notch 103 is disposed in the both side ends of the first processed part 101. In the embodiment, as illustrated in FIGS. 2A and 2B, the first notch 103 has a configuration including a first right notch 103a that is disposed in the right side end of the first processed part 101, and a first left notch 103b that is disposed in the left side end of the first processed part 101. Each of the notches has an approximately semicircular shape.

The second notch 104 is disposed in the both side ends of the second processed part 102. In the embodiment, as illustrated in FIGS. 2A and 2B, the second notch 104 has a configuration including a second right notch 104a that is disposed in the right side end of the second processed part 102, and a second left notch 104b that is disposed in the left side end of the second processed part 102. Each of the notches has an approximately semicircular shape.

Since the first notch 103 is disposed, the width of the first processed part 101 is smaller than that of the release sheet 100. In the same or similar manner, since the second notch 104 is disposed, the width of the second processed part 102 is smaller than that of the release sheet 100. Because of these, a situation can be prevented from occurring where, when the upper sheet portion 100a and the intermediate sheet portion 100b are to be separated from each other along the first processed part 101, or when the intermediate sheet portion 100b and the lower sheet portion 100c are to be separated from each other along the second processed part 102, the first connection portion 2d or the second connection portion 2e is stretched without slack during the course of the separation, and the release sheet 100 cannot be cut off up to the end.

The shape of the first notch 103, and that of the second notch 104 are not limited to an approximately semicircular shape, and may be any shape in so far as it can make the width of the first processed part 101 smaller than that of the release sheet 100, and that of the second processed part 102 smaller than that of the release sheet 100.

The rounded parts 105 are disposed in end parts of the first notch 103 and the second notch 104. Since the rounded parts 105 are disposed, here, a situation can be prevented from occurring where, when the release sheet 100 is to be cut off along the first processed part 101, or when the release sheet 100 is to be cut off along the second processed part 102, sharp edges are formed, and someone is injured.

The positioning hole 106 is provided to fix the release sheet 100 during a process for producing the body electrode unit U.

The patterned portions 107 are provided linearly in the longitudinal direction of the release sheet 100. Since the patterned portions 107 are disposed, the release sheet 100 can be easily recognized when it is transparent and is dropped on the floor. Therefore, it is possible to prevent someone from slipping by stepping on the release sheet 100. The patterned portions 107 may have any pattern in so far as the pattern enables the release sheet 100 to be easily recognized.

### Lateral Side of Body Electrode Unit U

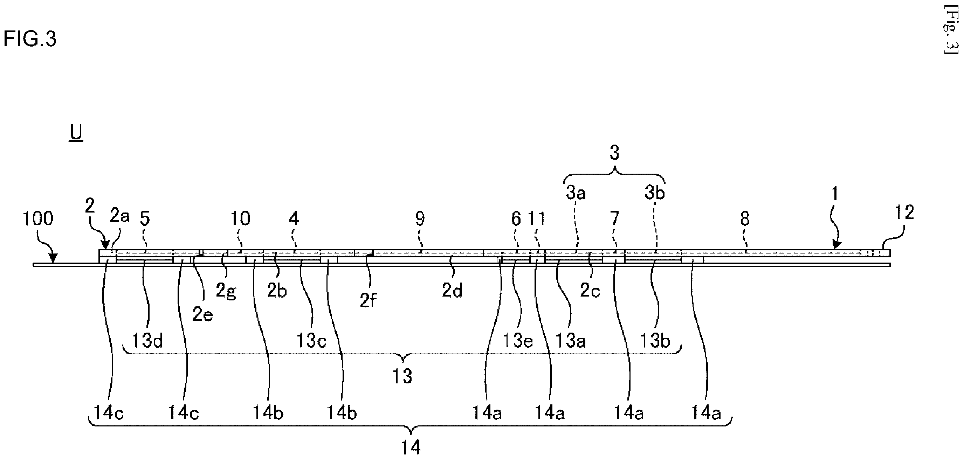

Referring to Fig. 3, next, a side view of the body electrode unit U will be described.

As illustrated in FIG. 3, the body electrode 1 is configured such that the stimulation electrode 3, the active electrode 4, and the reference electrode 5 are put together to form a single continuous shape. Specifically, the intermediate island portion 2b where the active electrode 4 is mounted, and the lower island portion 2c where the stimulation electrode 3 is mounted are connected to each other by the first connection portion 2d. The upper island portion 2a where the reference electrode 5 is mounted, and the intermediate island portion 2b where the active electrode 4 is mounted are connected to each other by the second connection portion 2e. This is how the stimulation electrode 3, the active electrode 4, and the reference electrode 5 are put together to form a single continuous shape in this example.

### Use of Body electrode Unit U

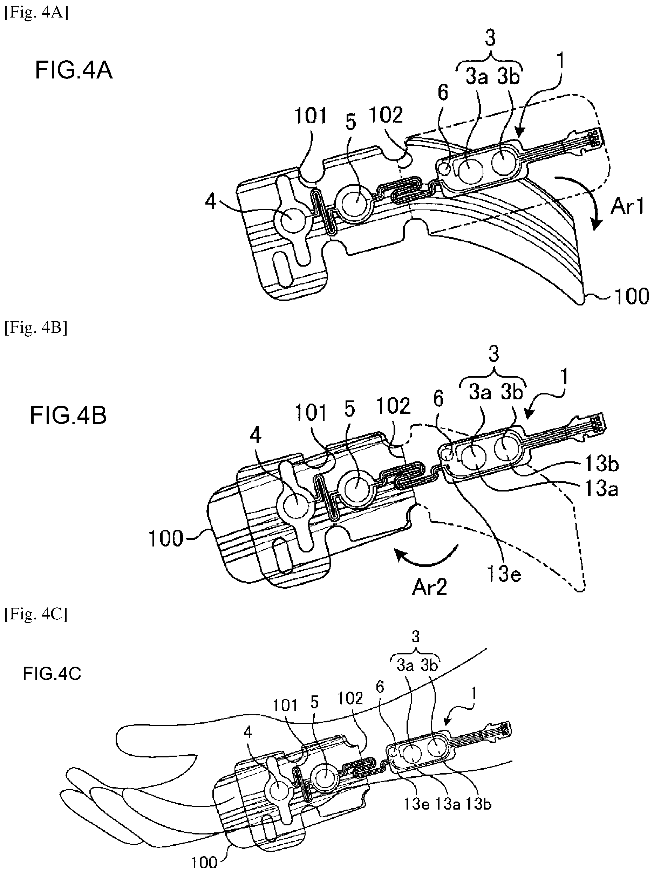

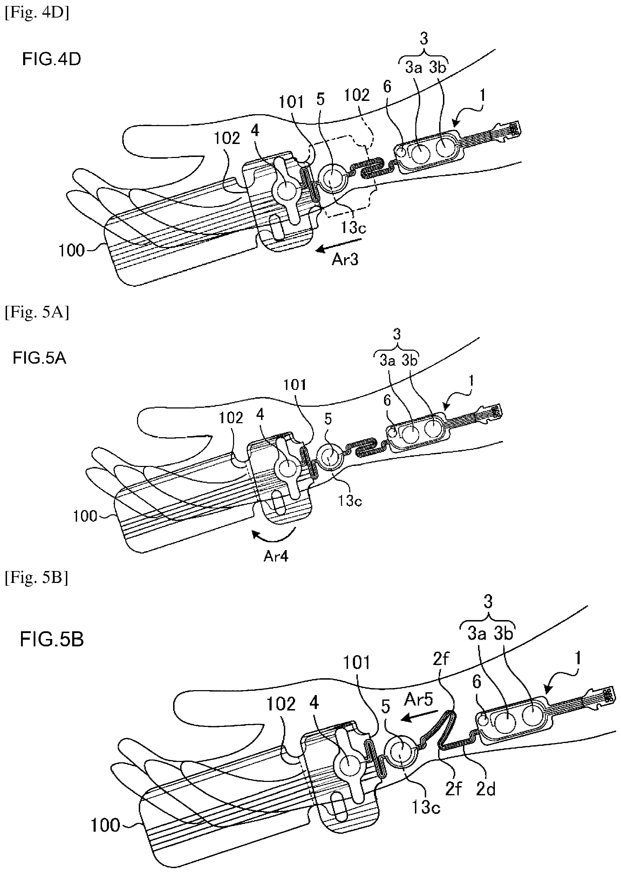

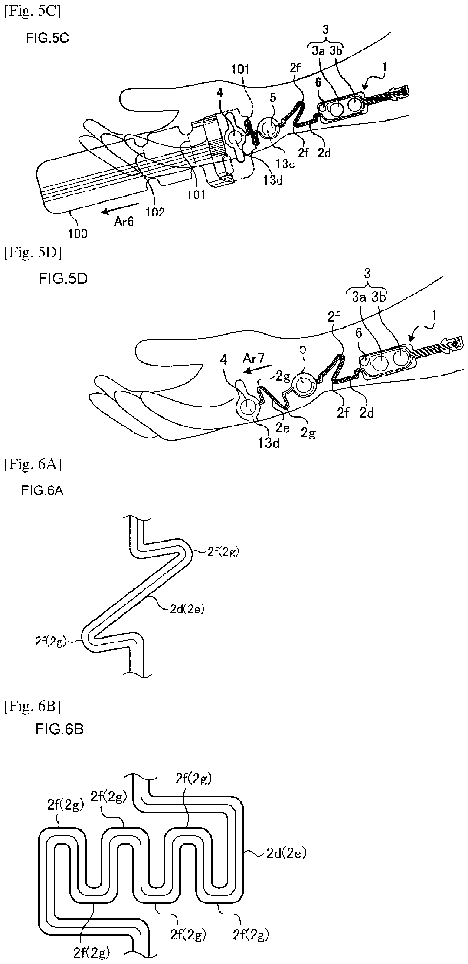

Referring to FIGS. 4 and 5, next, a use mode of the body electrode unit U will be described.

As described above, the body electrode 1 is attached to the release sheet 100. In the case where the body electrode 1 is to be used, therefore, the release sheet 100 is first moved in the direction of the arrow Ar1 as illustrated in FIG. 4A, whereby the attaching between the lower sheet portion 100c and the stimulation electrode adhesive tape 14a is released. As a result, the release sheet 100 is peeled off in a range extending to the second processed part 102.

As illustrated in FIG. 4B, next, the release sheet 100 is moved in the direction of the arrow Ar2, thereby bending the release sheet 100 along the second processed part 102. In the case where the second processed part 102 is configured by tearable perforations, although not illustrated, the lower sheet portion 100c may be separated from the release sheet 100 along the second processed part 102.

As illustrated in FIG. 4C, next, the stimulation electrode adhesive tape 14a is attached so that the negative electrode adhesive gel 13a and the positive electrode adhesive gel 13b correspond to the ulnar nerve of the subject.

As illustrated in FIG. 4D, next, the release sheet 100 is moved in the direction of the arrow Ar3, whereby the attaching between the intermediate sheet portion 100b and the active electrode adhesive tape 14b is released. As a result, the release sheet 100 is peeled off in a range extending to the first processed part 101.

As illustrated in FIG. 5A, next, the release sheet 100 is moved in the direction of the arrow Ar4, thereby bending the release sheet 100 along the first processed part 101. In the case where the first processed part 101 is configured by tearable perforations, although not illustrated, the intermediate sheet portion 100b may be separated from the upper sheet portion 100a along the first processed part 101.

As illustrated in FIG. 5B, next, the active electrode adhesive tape 14b is attached so that the active electrode adhesive gel 13c corresponds to the abductor digiti minimi muscle of the subject.

At this time, the first direction changing part 2f that can change at least one of the distance and angle between the stimulation electrode 3 and the active electrode 4 is disposed in the first connection portion 2d, and therefore the first connection portion 2d elongates in the direction of the arrow Ar5.

As illustrated in FIG. 5C, next, the release sheet 100 is moved in the direction of the arrow Ar6, whereby the attaching between the upper sheet portion 100a and the reference electrode adhesive tape 14c is released. As a result, the release sheet 100 is peeled off from the body electrode 1.

As illustrated in FIG. 5D, next, the reference electrode adhesive tape 14c is attached so that the reference electrode adhesive gel 13d corresponds to the abductor digiti minimi muscle of the subject.

At this time, the second direction changing part 2g that can change at least the distance and angle between the active electrode 4 and the reference electrode 5 is disposed in the second connection portion 2e, and therefore the second connection portion 2e elongates in the direction of the arrow Ar7.

As described above, the first direction changing part 2f is disposed in the first connection portion 2d in the embodiment, and therefore the first connection portion 2d can elongate. Moreover, the second direction changing part 2g is disposed in the second connection portion 2e in the embodiment, and therefore the second connection portion 2e can elongate. Because of these, in the body electrode 1 in which the stimulation electrode 3, the active electrode 4, and the reference electrode 5 are put together to form a single continuous shape, even in the case where the subject has a large hand, the lower island portion 2c where the stimulation electrode 3 is mounted, the intermediate island portion 2b where the active electrode 4 is mounted, and the upper island portion 2a where the reference electrode 5 is mounted can be attached to desired positions respectively.

Since the first connection portion 2d can elongate, and the second connection portion 2e can elongate, the body electrode 1 of the embodiment can be used not only while the lower island portion 2c where the stimulation electrode 3 is mounted, the intermediate island portion 2b where the active electrode 4 is mounted, and the upper island portion 2a where the reference electrode 5 is mounted are attached to the hand or arm of the subject, but also while these portions are attached to the foot of the subject.

On the other hand, also in the case where the body electrode 1 in which the stimulation electrode 3, the active electrode 4, and the reference electrode 5 are put together to form a single continuous shape is used in a subject having a small hand, there is a problem in that the stimulation electrode 3, the active electrode 4, and the reference electrode 5 are hardly attached to desired positions.

In the embodiment, by contrast, the first direction changing part 2f is disposed in the first connection portion 2d, and therefore the first connection portion 2d can be shortened. In the embodiment, furthermore, the second direction changing part 2g is disposed in the second connection portion 2e, and therefore the second connection portion 2e can be shortened. Because of these, in the body electrode 1 in which the stimulation electrode 3, the active electrode 4, and the reference electrode 5 put together to form a single continuous shape, even in the case where the subject has a small hand, the lower island portion 2c where the stimulation electrode 3 is mounted, the intermediate island portion 2b where the active electrode 4 is mounted, and the upper island portion 2a where the reference electrode 5 is mounted can be attached to desired positions.

In the case where the subject has a small hand, when the first connection portion 2d is shortened by the first direction changing part 2f, the negative electrode 3a configured by a cathode, and the active electrode 4 configured by a cathode are close to each other. Therefore, it may be considered that a detected physiological signal is made unstable by noises. However, the neutral electrode 6 is disposed proximal to the negative electrode 3a. Even in the case where the negative electrode 3a and the active electrode 4 are close to each other, therefore, noises can be efficiently blocked, and hence a detected physiological signal can be stabilized.

### Examples of First Connection Portion 2d and Second Connection Portion 2e

Referring to FIGS. 6A to 6C, other examples of the first connection portion 2d and the second connection portion 2e will be described.

FIG. 6A illustrates an example of the first connection portion 2d and the second connection portion 2e. In this example, in the first connection portion 2d and the second connection portion 2e, each of the first direction changing part 2f and the second direction changing part 2g is disposed at two locations, and are configured to change the directions in which the first connection portion 2d and the second connection portion 2e extend in oblique directions.

FIG. 6B illustrates another example of the first connection portion 2d and the second connection portion 2e. In this example, in the first connection portion 2d and the second connection portion 2e, each of the first direction changing part 2f and the second direction changing part 2g is disposed at six locations, and are configured to change the first connection portion 2d and the second connection portion 2e in the longitudinal direction of the body electrode.

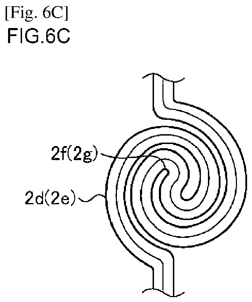

FIG. 6C illustrates another example of the first connection portion 2d and the second connection portion 2e. In this example, in the first connection portion 2d and the second connection portion 2e, each of the first direction changing part 2f and the second direction changing part 2g is disposed at one location, and are configured to change the direction in which the first connection portion 2d and the second connection portion 2ec extend in a rotational direction.

As described above, the direction in which the first connection portion 2d is changed by the first direction changing part 2f, and the direction in which the second connection portion 2e is changed by the second direction changing part 2g can be adequately set.

The number of the first direction changing part 2f that is provided in the first connection portion 2d is required to be at least one, and may be adequately set.

In the same or similar manner, the number of the second direction changing part 2g that is provided in the second connection portion 2e is required to be at least one, and may be adequately set.

### Another Example of Neutral Electrode 6

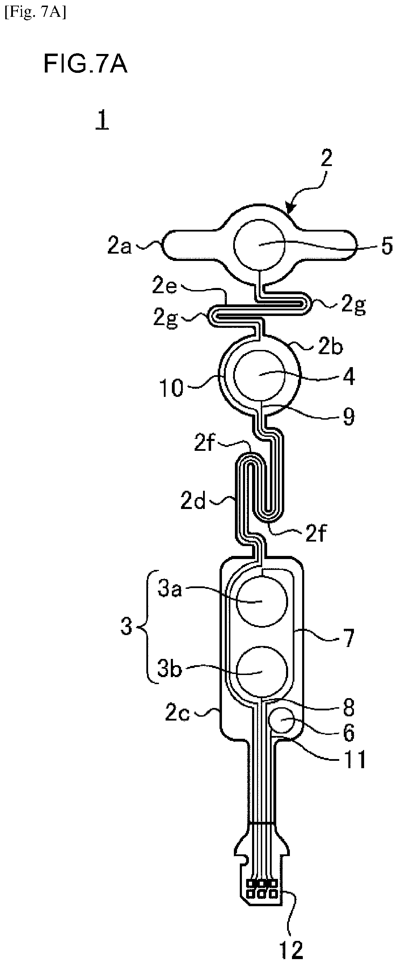

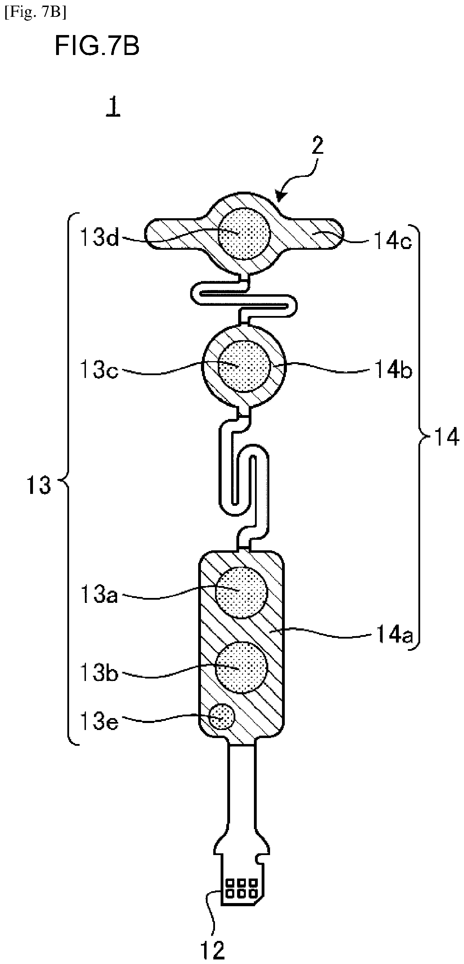

Referring to FIGS. 7A and 7B, next, another example of the neutral electrode 6 will be described.

In the above-described embodiment, the neutral electrode 6 is disposed proximal to the negative electrode 3a. However, the position where the neutral electrode 6 is disposed can be adequately set in so far as noises flowing through the body electrode 1 can be blocked. Hereinafter, a preferred example of the position where the neutral electrode 6 is disposed will be described.

As illustrated in FIGS. 7A and 7B, the neutral electrode 6 of this example is disposed at a position that is closer to the connector 12 as compared with the stimulation electrode 3. The distance with respect to the connector 12 is short, and therefore the neutral electrode wiring part 11 can be shortened. Consequently, the production cost can be reduced.

In the case where the neutral electrode 6 is disposed in the upper island portion 2a or the intermediate island portion 2b, a part of the neutral electrode wiring part 11 must be disposed in the first connection portion 2d or the second connection portion 2e, and therefore it is required to thicken the first connection portion 2d or the second connection portion 2e. As a result, the production cost is raised by an amount corresponding to the thickened part of the first connection portion 2d or the second connection portion 2e. As compared with the case where the neutral electrode 6 is disposed in the upper island portion 2a or the intermediate island portion 2b, therefore, the production cost can be reduced.

### Potential Waveform of Active Electrode 4

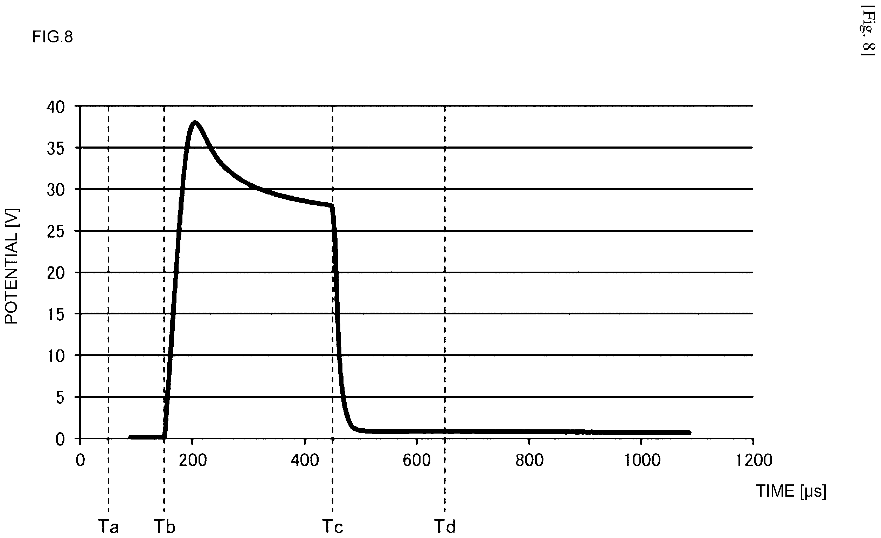

Next, the potential waveform of the active electrode 4 will be described with reference to FIG. 8.

First, the timing Ta in FIG. 8 is a timing when a stimulation current has not flown through the body electrode 1. Here, although impedances exist among the stimulation electrode 3, the active electrode 4, and the reference electrode 5, the stimulation current does not flow through the body electrode 1, and therefore there is no potential difference among the stimulation electrode 3, the active electrode 4, and the reference electrode 5.

Next, the timing Tb in FIG. 8 is a timing when the stimulation current begins to flow through the body electrode 1. When the stimulation current flows through the body electrode 1, a potential difference is produced between the negative electrode 3a and the positive electrode 3b. Specifically, an impedance having a value that is a product of the stimulation current flowing through the body electrode 1, and the impedance between the negative electrode 3a and the positive electrode 3b is produced.

When the body electrode 1 is attached to the living body as illustrated in FIG. 5D, potential differences are produced also between the negative electrode 3a and the active electrode 4, and between the negative electrode 3a and the reference electrode 5 through the impedances in and under the skin. The potential differences are produced during the time period when the stimulation current is caused to flow (specifically, the time period between the timing Tb and the timing Tc).

Next, the timing Tc in FIG. 8 is a timing when the stimulation current stops to flow through the body electrode 1. When the flow of the stimulation current through the body electrode 1 is ended, electric charges are discharged from the active electrode 4 and the reference electrode 5 toward the negative electrode 3a.

Next, the timing Td in FIG. 8 is a timing when the detection of artifact noise is started. The shorter the discharging time period when electric charges are discharged from the active electrode 4 and the reference electrode 5 toward the negative electrode 3a, the lower the level of artifact noise.

The rate of the discharge is inversely proportional to a CR time constant that is a product of the capacitance C and the resistance R. When the neutral electrode 6 is mounted proximal to the active electrode 4 and the reference electrode 5, therefore, the charges are separated into charges that are discharged from the active electrode 4 to the negative electrode 3a through the living body, and those that are discharged from the neutral electrode 6 to the negative electrode 3a through the active electrode 4 and the internal circuit of the electric stimulator.

The impedances have a positive correlation with the distance on the surface of the living body. When the neutral electrode 6 is mounted in a place remote from the negative electrode 3a, such as the position of the neutral electrode 6 in the example of FIGS. 7A and 7B, therefore, the impedance between the neutral electrode 6 and the negative electrode 3a is increased. Therefore, the amount of charges that are discharged through the internal circuit of the electric stimulator is reduced, and the rate of discharge becomes low, with the result that the level of artifact noise is increased. Because of these, the neutral electrode 6 is preferably mounted proximal to the negative electrode 3a from the view point of the reduction of artifact noise.

When the neutral electrode 6 is mounted on a lower side of the negative electrode 3a and the positive electrode 3b, the charges are hardly separated into charges that are discharged from the active electrode 4 to the negative electrode 3a through the living body, and charges that are discharged from the neutral electrode 6 to the negative electrode 3a through the active electrode 4 and the internal circuit of the electric stimulator. It is desirable from this viewpoint that the neutral electrode 6 be mounted on an upper side of the negative electrode 3a and the positive electrode 3b than being mounted on the lower side of the negative electrode 3a and the positive electrode 3b.

An electromyogram is measured after the timing Td. Specifically, the measurement of an electromyogram is started at a first timing that is after 1,200 µs, and ended at a second timing that is after the first timing.

### Another Example of Neutral Electrode 6

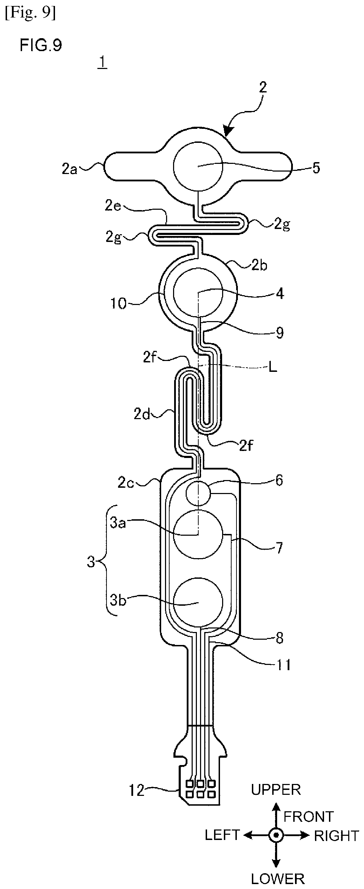

Next, another example of the neutral electrode 6 will be described with reference to FIG. 9.

As illustrated in FIG. 9, the neutral electrode 6 of this example is mounted in the lower island portion 2c. Moreover, the neutral electrode 6 is mounted on the upper side of the negative electrode 3a. As compared with the position where the neutral electrode 6 of FIGS. 7A and 7B is mounted, the neutral electrode 6 is mounted proximal to the negative electrode 3a.

As compared with the case where the neutral electrode 6 is mounted on the lower side of the negative electrode 3a and the positive electrode 3b, therefore, the impedance between the neutral electrode 6 and the negative electrode 3a is reduced. The amount of charges that are discharged through the internal circuit of the electric stimulator is increased, and the rate of discharge becomes high, with the result that artifact noise can be reduced.

The neutral electrode 6 of this example is mounted on a line segment L connecting the center of the negative electrode 3a and the center of the active electrode 4. As compared with the body electrode 1 of the example of FIGS. 1A and 1B, therefore, the line connecting the center of the negative electrode 3a and the center of the neutral electrode 6 is shortened, and consequently the neutral electrode 6 is proximal to the negative electrode 3a.

As compared with the case where the neutral electrode 6 is not mounted on the line segment L connecting the center of the negative electrode 3a and the center of the active electrode 4, therefore, the impedance between the neutral electrode 6 and the negative electrode 3a is reduced. The amount of charges that are discharged through the internal circuit of the electric stimulator is increased, and the rate of discharge becomes high, with the result that artifact noise can be reduced.

### Another Example of Neutral Electrode 6

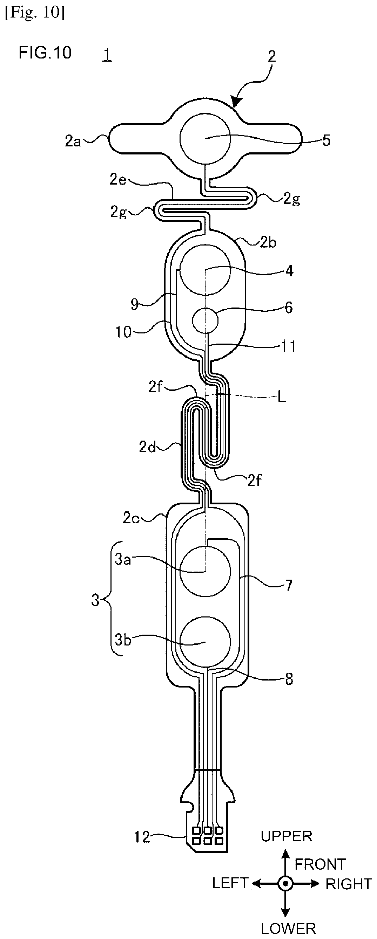

Next, another example of the neutral electrode 6 will be described with reference to FIG. 10.

As illustrated in FIG. 10, the neutral electrode 6 of this example is mounted on the upper side of the negative electrode 3a and in the intermediate island portion 2b. The neutral electrode 6 is mounted on the line segment L connecting the center of the negative electrode 3a and the center of the active electrode 4.

As compared with the case where the neutral electrode 6 is mounted in the intermediate island portion 2b and in a place other than that on the line segment L, therefore, the line connecting the center of the negative electrode 3a and the center of the neutral electrode 6 is shortened, and consequently the neutral electrode 6 is proximal to the negative electrode 3a.

Therefore, the impedance between the neutral electrode 6 and the negative electrode 3a is reduced. The amount of charges that are discharged through the internal circuit of the electric stimulator is increased, and the rate of discharge becomes high, with the result that artifact noise can be reduced.

As indicated in the examples of FIGS. 9 and 10, when the neutral electrode 6 is mounted on the upper side of the negative electrode 3a and the positive electrode 3b, and on the line segment L, artifact noise can be efficiently reduced as compared with the case where the neutral electrode 6 is mounted at another position.

### Another Example of Neutral Electrode 6

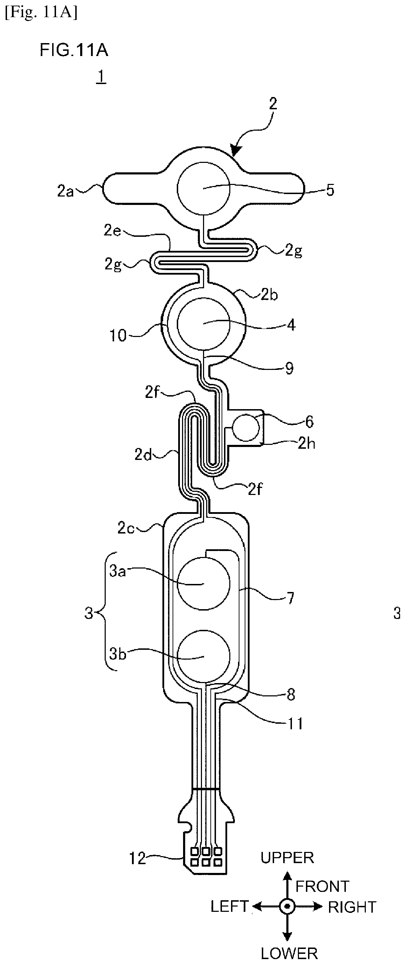

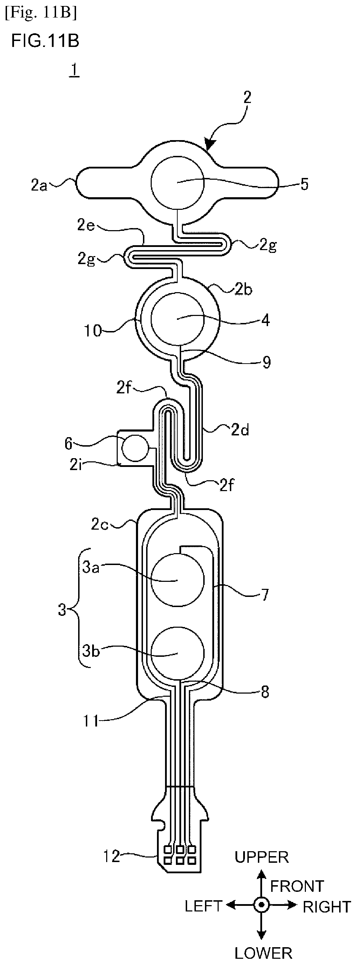

Next, another example of the neutral electrode 6 will be described with reference to FIGS. 11A and 11B.

The body electrode 1 of FIG. 11A has a right island portion 2h disposed on the right side of the first connection portion 2d. The neutral electrode 6 is mounted in the right island portion 2h. In other words, the neutral electrode 6 is mounted on an upper side of the negative electrode 3a. According to this configuration, artifact noise that is produced in the body electrode 1 can be reduced. In the body electrode 1 of the fourth modification, the neutral electrode 6 is mounted in the right island portion 2h that is located between the negative electrode 3a and the active electrode 4, and therefore artifact noise that is produced in the body electrode 1 can be reduced as compared with the case where the neutral electrode 6 is mounted in a region other than the region between the negative electrode 3a and the active electrode 4.

As illustrated in FIG. 11B, alternatively, a left island portion 2i may be disposed on the left side of the first connection portion 2d, and the neutral electrode 6 may be mounted in the left island portion 2i. Also in this case, in the same or similar manner as the body electrode 1 illustrated in FIG. 11A, the neutral electrode 6 is mounted on the upper side of the negative electrode 3a, and therefore artifact noise that is produced in the body electrode 1 can be reduced. Also in the case where the neutral electrode 6 is mounted in the left island portion 2i, it can be said that the neutral electrode 6 is mounted in the region between the negative electrode 3a and the active electrode 4, and therefore artifact noise that is produced in the body electrode 1 can be reduced as compared with the case where the neutral electrode 6 is mounted in a region other than the region between the negative electrode 3a and the active electrode 4.

### Other Examples

Other examples will be described below.

In the body electrode 1 of the above-described embodiment, the stimulation electrode 3, the active electrode 4, and the reference electrode 5 are mounted so that a straight line connects their centers. However, the stimulation electrode 3, the active electrode 4, and the reference electrode 5 may be mounted such that they are deviated in the lateral direction of the body electrode 1.

In the body electrode 1 of the above-described embodiment, the reference electrode 5 is mounted in the upper island portion 2a, and the active electrode 4 is mounted in the intermediate island portion 2b. Alternatively, the active electrode 4 may be mounted on the upper island portion 2a, and the reference electrode 5 may be mounted on the intermediate island portion 2b.

In the body electrode 1 of the above-described embodiment, the negative electrode 3a is disposed at a position that is remoter from the connector 12 with respect to the positive electrode 3b, and the positive electrode 3b is disposed at a position that is closer to the connector 12 with respect to the negative electrode 3a. Alternatively, the position where the positive electrode 3b is mounted and the position where the negative electrode 3a is mounted may be reversed.

Although, in the above-described embodiment, the first direction changing part 2f changes the direction in which the first connection portion 2d extends in the longitudinal direction of the body electrode 1, the direction in which the first connection portion 2d extends may be changed in the lateral direction of the body electrode 1. Likewise, although the second direction changing part 2g changes the direction in which the second connection portion 2e extends in the lateral direction, the direction in which the second connection portion 2e extends may be changed in the longitudinal direction.

Although, in the above-described embodiment, the first direction changing part 2f and the second direction changing part 2g change the directions of the first connection portion 2d and the second connection portion 2e in different directions, respectively, the directions of the first connection portion 2d and the second connection portion 2e may be changed in the same direction.

Although, in the above-described embodiment, a perforation process and a half-cut process have been described as specific examples of the first processed part 101 and the second processed part 102, the presently disclosed subject matter is not limited to this. For example, a spot fixing process in which the both ends of the first processed part 101 and the second processed part 102 are spot-fixed, and the spot-fixed ends are separated from each other may be applied. Alternatively, a cut may be provided at least one side of the first processed part 101 and the second processed part 102.

Although, in the above-described embodiment, the first processed part 101 and the second processed part 102 employ the same process as a mode in which the release sheet 100 can be separated and/or bent, the parts may employ different processes. For example, the first processed part 101 may employ a perforation process, and the second processed part 102 may employ a half-cut process.

Although, in the above-described embodiment, the first processed part 101 and the second processed part 102 enable the release sheet 100 to be separated and/or bent in a horizontal direction, the presently disclosed subject matter is not limited to this. The release sheet 100 may be separated and/or bent in any direction. For example, the first processed part 101 and the second processed part 102 may have a mode in which the release sheet 100 can be separated and/or bent in an oblique direction.

Although, in the above-described embodiment, the first notch 103 and the second notch 104 are disposed in the respective both side ends, the presently disclosed subject matter is not limited to this. The first notch 103 and the second notch 104 may be disposed in only one side, i.e., the right side end or the left side end.

Although, in the above-described embodiment, the electrodes and the electric stimulator are connected to each other through the respective wiring parts, the electrodes and the electric stimulator may be connected to each other through a wireless function.

The way the body electrode unit U is used is not limited to the example illustrated in FIGS. 4A to 5D. That is, as described in the following examples, the body electrode unit U may be used in different ways. Thus, it is possible to provide options for a medical person in using the body electrode unit U.

According to a first example, first, the lower sheet portion 100c is separated along the first processed part 101, the lower island portion 2c is peeled off from the lower sheet portion 100c, and the stimulation electrode adhesive tape 14a is attached to the subject. Next, the intermediate sheet portion 100b is separated along the second processed part 102, the intermediate island portion 2b is peeled off from the intermediate sheet portion 100b, and the active electrode adhesive tape 14b is attached to the subject. Then, the upper island portion 2a is peeled off from the upper sheet portion 100a, and the reference electrode adhesive tape 14c is attached to the subject.

According to a second example, first, the upper island portion 2a is peeled off from the upper sheet portion 100a, the release sheet 100 is bent along the second processed part 102, and the reference electrode adhesive tape 14c is attached to the subject. Next, the intermediate island portion 2b is peeled off from the intermediate sheet portion 100b, the release sheet 100 is bent along the first processed part 101, and the active electrode adhesive tape 14b is attached to the subject. Then, the lower island portion 2c is peeled off from the lower sheet portion 100c, and the stimulation electrode adhesive tape 14a is attached to the subject.

According to a third example, first, the upper sheet portion 100a is separated along the second processed part 102, the upper island portion 2a is peeled off from the upper sheet portion 100a, and the reference electrode adhesive tape 14c is attached to the subject. Next, the intermediate sheet portion 100b is separated along the first processed part 101, the intermediate island portion 2b is peeled off from the intermediate sheet portion 100b, and the active electrode adhesive tape 14b is attached to the subject. Then, the lower island portion 2c is peeled off from the lower sheet portion 100c, and the stimulation electrode adhesive tape 14a is attached to the subject.

As described above, the body electrode 1 includes the stimulation electrode 3, the active electrode 4, and the reference electrode 5. The first connection portion 2d is disposed between the stimulation electrode 3 and the active electrode 4, and the second connection portion 2e is disposed between the active electrode 4 and the reference electrode 5. The first connection portion 2d has the first direction changing part 2f configured to change the direction in which the first connection portion 2d extends, so that at least one of the distance and the angle between the stimulation electrode 3 and the active electrode 4 is adjustable. According to the configuration, the first connection portion 2d can be elongated and shortened. Therefore, it is possible to provide a body electrode and body electrode unit in which an electrode can be attached to a desired position.

According to an aspect of the embodiments described above, a body electrode includes a first electrode (for example, the stimulation electrode 3) configured to stimulate a muscle of a body, a second electrode (for example, the active electrode 4) and a third electrode (for example, the reference electrode 5) configured to detect a physiological signal from the muscle that is stimulated by the first electrode, a first connection portion (for example, the first connection portion 2d) arranged between the first electrode and the second electrode, and a second connection portion (for example, the second connection portion 2e) arranged between the third electrode and one of the first electrode and the second electrode. The first connection portion has at least one first direction changing part (for example, the first direction changing part 2f) configured to change a direction in which the first connection portion extends, such that at least one of a distance and an angle between the first electrode and the second electrode is adjustable. In other words, the at least one first direction changing part is configured to allow a movement of the second electrode relative to the first electrode, the movement of the second electrode including at least one of a linear movement and a rotational movement.

According to another aspect of the embodiments described above, the second connection portion may have at least one second direction changing part (for example, the second direction changing parts 2g) configured to change a direction in which the second connection portion extends, such that at least one of a distance and an angle between the third electrode and the one of the first electrode and the second electrode is adjustable. In other words, the at least one second direction changing part is configured to allow a movement of the third electrode relative to the one of the first electrode and the second electrode, the movement of the third electrode including at least one of a linear movement and a rotational movement.

According to another aspect of the embodiments described above, the first electrode, the second electrode, and the third electrode may be arranged in a row.

According to another aspect of the embodiments described above, the first direction changing part and the second direction changing part may be configured to change the direction in which the first connection portion extends and the direction in which the second connection portion extends in different directions.

According to another aspect of the embodiments described above, the first direction changing part may be configured to change the direction in which the first connection portion extends in a longitudinal direction of the body electrode, and the second direction changing part may be configured to change the direction in which the second connection portion extends in a lateral direction of the body electrode.

According to another aspect of the embodiments described above, the body electrode may further include a first electrode mounting portion (for example, the lower island portion 2c) having an adhesiveness and on which the first electrode is mounted, a second electrode mounting portion (for example, the intermediate island portion 2b) having an adhesiveness and on which the second electrode is mounted, and a third electrode mounting portion (for example, the upper island portion 2a) having an adhesiveness and on which the third electrode is mounted. The first connection portion connects the first electrode mounting portion and the second electrode mounting portion to each other, and does not have an adhesiveness. The second connection portion connects the second electrode mounting portion and the third electrode mounting portion to each other, and does not have an adhesiveness.

According to another aspect of the embodiments described above, the first electrode mounting portion may include a neutral electrode (for example, the neutral electrode 6) configured to eliminate a noise flowing through the body electrode.

According to another aspect of the embodiments described above, the first electrode may include a negative electrode (for example, the negative electrode 3a) and a positive electrode (for example, the positive electrode 3b), and the neutral electrode may be mounted closer to the negative electrode than to the positive electrode.

According to another aspect of the embodiments described above, the first electrode may include a negative electrode (for example, the negative electrode 3a), and a positive electrode (for example, the positive electrode 3b), and the neutral electrode may be mounted on an upper side of the negative electrode.

According to another aspect of the embodiments described above, the neutral electrode may be mounted between the negative electrode and the second electrode.

According to another aspect of the embodiments described above, the neutral electrode may be mounted on a line segment (for example, the line segment L) connecting the center of the negative electrode and the center of the second electrode.

According to another aspect of the embodiments described above, a body electrode unit includes the body electrode (for example, the body electrode 1), and a release sheet (for example, the release sheet 100) to which the body electrode is attached.

The invention is set out in the independent claim 1. Preferred embodiments are set out in the dependent claims.

## Claims

1. A body electrode (1) comprising:
a first electrode (3) configured to stimulate a muscle of a body;
a second electrode (4) and a third electrode (5), the second electrode and the third electrode being configured to detect a physiological signal from the muscle that is stimulated by the first electrode;
a first connection portion (2d) arranged between the first electrode and the second electrode; and
a second connection (2e) portion arranged between the third electrode and one of the first electrode and the second electrode,
wherein the first connection portion comprises at least one first direction changing part (2f) configured to change a direction in which the first connection portion extends in a longitudinal direction of the body electrode, such that a distance and an angle between the first electrode and the second electrode is adjustable.

2. The body electrode according to claim 1, wherein the second connection portion comprises at least one second direction changing part (2g) configured to change a direction in which the second connection portion extends, such that at least one of a distance and an angle between the third electrode and the one of the first electrode and the second electrode is adjustable.

3. The body electrode according to claim 1 or 2, wherein the first electrode, the second electrode, and the third electrode are arranged in a row.

4. The body electrode according to claim 2, wherein the first direction changing part and the second direction changing part are configured to change the direction in which the first connection portion extends and the direction in which the second connection portion extends in different directions.

5. The body electrode according to claim 4, wherein the first direction changing part is configured to change the direction in which the first connection portion extends in a longitudinal direction of the body electrode, and
wherein the second direction changing part is configured to change the direction in which the second connection portion extends in a lateral direction of the body electrode.

6. The body electrode according to any one of claims 1 to 5, further comprising:
a first electrode mounting portion (2c) on which the first electrode is mounted, the first electrode mounting portion being adhesive;
a second electrode mounting portion (2b) on which the second electrode is mounted, the second electrode mounting portion being adhesive; and
a third electrode mounting portion (2a) on which the third electrode is mounted, the third electrode mounting portion being adhesive,
wherein the first connection portion connects the first electrode mounting portion and the second electrode mounting portion to each other, the first connection portion being non-adhesiveness, and
wherein the second connection portion connects the second electrode mounting portion and the third electrode mounting portion to each other, the second connection portion being non-adhesive.

7. The body electrode according to claim 6, wherein the first electrode mounting portion comprises a neutral electrode (6) configured to eliminate a noise flowing through the body electrode.

8. The body electrode according to claim 7, wherein the first electrode comprises a negative electrode (3a) and a positive electrode (3b), the neutral electrode being closer to the negative electrode than to the positive electrode.

9. The body electrode according to claim 7, wherein the first electrode comprises a negative electrode and a positive electrode, the neutral electrode being mounted on an upper side of the negative electrode.

10. The body electrode according to claim 9, wherein the neutral electrode is mounted between the negative electrode and the second electrode.

11. The body electrode according to claim 9 or 10, wherein the neutral electrode is mounted on a line segment connecting a center of the negative electrode and a center of the second electrode.

12. A body electrode unit comprising:
the body electrode according to any one of claims 1 to 11; and
a release sheet (100) to which the body electrode is attached.

## Patentansprüche

1. Körperelektrode (1), die umfasst:
eine erste Elektrode (3), die so ausgeführt ist, dass sie einen Muskel eines Körpers stimuliert;
eine zweite Elektrode (4) sowie eine dritte Elektrode (5), wobei die zweite Elektrode und die dritte Elektrode so ausgeführt sind, dass sie ein physiologisches Signal von dem Muskel erfassen, der durch die erste Elektrode stimuliert wird;
einen ersten Verbindungsabschnitt (2d), der zwischen der ersten Elektrode und der zweiten Elektrode angeordnet ist; sowie
einen zweiten Verbindungsabschnitt (2e), der zwischen der dritten Elektrode und der ersten oder der zweiten Elektrode angeordnet ist,
wobei der erste Verbindungsabschnitt wenigstens einen ersten Richtungsänderungsteil (2f) umfasst, der so ausgeführt ist, dass er eine Richtung, in der sich der erste Verbindungsabschnitt in einer Längsrichtung der Körperelektrode erstreckt, so ändert, dass ein Abstand und ein Winkel zwischen der ersten Elektrode und der zweiten Elektrode angepasst werden können.

2. Körperelektrode nach Anspruch 1, wobei der zweite Verbindungsabschnitt wenigstens einen zweiten Richtungsänderungsteil (2g) umfasst, der so ausgeführt ist, dass er eine Richtung, in der sich der zweite Verbindungsabschnitt erstreckt, so ändert, dass ein Abstand oder/und ein Winkel zwischen der dritten Elektrode und der ersten oder der zweiten Elektrode angepasst werden kann.

3. Körperelektrode nach Anspruch 1 oder 2, wobei die erste Elektrode, die zweite Elektrode und die dritte Elektrode in einer Reihe angeordnet sind.

4. Körperelektrode nach Anspruch 2, wobei der erste Richtungsänderungsteil und der zweite Richtungsänderungsteil so ausgeführt sind, dass sie die Richtung, in der sich der erste Verbindungsabschnitt erstreckt, und die Richtung, in der sich der zweite Verbindungsabschnitt erstreckt, zu unterschiedlichen Richtungen ändern.

5. Körperelektrode nach Anspruch 4, wobei der erste Richtungsänderungsteil so ausgeführt ist, dass er die Richtung ändert, in der sich der erste Verbindungsabschnitt in einer Längsrichtung der Körperelektrode erstreckt, und
der zweite Richtungsänderungsteil so ausgeführt ist, dass er die Richtung ändert, in der sich der zweite Verbindungsabschnitt in einer Querrichtung der Körperelektrode erstreckt.

6. Körperelektrode nach einem der Ansprüche 1 bis 5, die des Weiteren umfasst:
einen ersten Elektroden-Anbringungsabschnitt (2c), an dem die erste Elektrode angebracht ist, wobei der erste Elektroden-Anbringungsabschnitt haftend ist;
einen zweiten Elektroden-Anbringungsabschnitt (2b), an dem die zweite Elektrode angebracht ist, wobei der zweite Elektroden-Anbringungsabschnitt haftend ist; sowie
einen dritten Elektroden-Anbringungsabschnitt (2a), an dem die dritte Elektrode angebracht ist, wobei der dritte Elektroden-Anbringungsabschnitt haftend ist,
wobei der erste Verbindungsabschnitt den ersten Elektroden-Anbringungsabschnitt und den zweiten Elektroden-Anbringungsabschnitt miteinander verbindet, und der erste Verbindungsabschnitt nicht haftend ist, und
wobei der zweite Verbindungsabschnitt den zweiten Elektroden-Anbringungsabschnitt und den dritten Elektroden-Anbringungsabschnitt miteinander verbindet, und der zweite Verbindungsabschnitt nicht haftend ist.

7. Körperelektrode nach Anspruch 6, wobei der erste Elektroden-Anbringungsabschnitt eine neutrale Elektrode (6) umfasst, die so ausgeführt ist, dass sie ein durch die Körperelektrode fließendes Rauschen eliminiert.

8. Körperelektrode nach Anspruch 7, wobei die erste Elektrode eine negative Elektrode (3a) sowie eine positive Elektrode (3b) umfasst und die neutrale Elektrode näher an der negativen Elektrode liegt als an der positiven Elektrode.

9. Körperelektrode nach Anspruch 7, wobei die erste Elektrode eine negative Elektrode sowie eine positive Elektrode umfasst und die neutrale Elektrode an einer Oberseite der negativen Elektrode angebracht ist.

10. Körperelektrode nach Anspruch 9, wobei die neutrale Elektrode zwischen der negativen Elektrode und der zweiten Elektrode angebracht ist.

11. Körperelektrode nach Anspruch 9 oder 10, wobei die neutrale Elektrode auf einer Strecke angebracht ist, die einen Mittelpunkt der negativen Elektrode und einen Mittelpunkt der zweiten Elektrode verbindet.

12. Körperelektroden-Einheit, die umfasst:
die Körperelektrode nach einem der Ansprüche 1 bis 11; sowie
eine Trennfolie (100), an der die Körperelektrode angebracht ist.

## Revendications

1. Électrode corporelle (1) comprenant :
une première électrode (3) configurée pour stimuler un muscle d'un corps ;
une seconde électrode (4) et une troisième électrode (5), la seconde électrode et la troisième électrode étant configurées pour détecter un signal physiologique depuis le muscle qui est stimulé par la première électrode ;
une première portion de connexion (2d) agencée entre la première électrode et la seconde électrode ; et
une seconde portion de connexion (2e) agencée entre la troisième électrode et l'une de la première électrode et de la seconde électrode,
la première portion de connexion comprenant au moins une première pièce de changement de sens (2f) configurée pour changer un sens dans lequel la première portion de connexion s'étend dans un sens longitudinal de l'électrode corporelle, de sorte qu'une distance et un angle entre la première électrode et la seconde électrode sont ajustables.

2. Électrode corporelle selon la revendication 1, la seconde portion de connexion comprenant au moins une seconde pièce de changement de sens (2g) configurée pour changer un sens dans lequel la seconde portion de connexion s'étend, de sorte qu'au moins une distance et un angle entre la troisième électrode et l'une de la première électrode et de la seconde électrode sont ajustables.

3. Électrode corporelle selon la revendication 1 ou 2, la première électrode, la seconde électrode, et la troisième électrode étant agencées en une rangée.

4. Électrode corporelle selon la revendication 2, la première pièce de changement de sens et la seconde pièce de changement de sens étant configurées pour changer le sens dans lequel la première portion de connexion s'étend et le sens dans lequel la seconde portion de connexion s'étend dans différents sens.

5. Électrode corporelle selon la revendication 4, la première pièce de changement de sens étant configurée pour changer le sens dans lequel la première portion de connexion s'étend dans un sens longitudinal de l'électrode corporelle, et
la seconde pièce de changement de sens étant configurée pour changer le sens dans lequel la seconde portion de connexion s'étend dans un sens latéral de l'électrode corporelle.

6. Électrode corporelle selon l'une quelconque des revendications 1 à 5, comprenant en outre :
une première portion de montage d'électrode (2c) sur laquelle la première électrode est montée, la première portion de montage d'électrode étant adhésive ;
une seconde portion de montage d'électrode (2b) sur laquelle la seconde électrode est montée, la seconde portion de montage d'électrode étant adhésive ; et
une troisième portion de montage d'électrode (2a) sur laquelle la troisième électrode est montée, la troisième portion de montage d'électrode étant adhésive,
la première portion de connexion connectant la première portion de montage d'électrode et la seconde portion de montage d'électrode l'une à l'autre, la première portion de connexion n'étant pas adhésive, et
la seconde portion de connexion connectant la seconde portion de montage d'électrode et la troisième portion de montage d'électrode l'une à l'autre, la seconde portion de connexion étant non adhésive.

7. Électrode corporelle selon la revendication 6, la première portion de montage d'électrode comprenant une électrode neutre (6) configurée pour éliminer un bruit s'écoulant à travers l'électrode corporelle.

8. Électrode corporelle selon la revendication 7, la première électrode comprenant une électrode négative (3a) et une électrode positive (3b), l'électrode neutre étant plus proche de l'électrode négative que de l'électrode positive.

9. Électrode corporelle selon la revendication 7, la première électrode comprenant une électrode négative et une électrode positive, l'électrode neutre étant montée sur un côté supérieur de l'électrode négative.

10. Électrode corporelle selon la revendication 9, l'électrode neutre étant montée entre l'électrode négative et la seconde électrode.

11. Électrode corporelle selon la revendication 9 ou 10, l'électrode neutre étant montée sur un segment linéaire connectant un centre de l'électrode négative et un centre de la seconde électrode.

12. Unité électrode corporelle comprenant :
l'électrode corporelle selon l'une quelconque des revendications 1 à 11 ; et
une feuille de décollement (100) à laquelle l'électrode corporelle est fixée.
